(19) Europäisches Patentamt
European Patent Office
Office européen des brevets

(11) **EP 4 572 590 A1**

(12) **EUROPEAN PATENT APPLICATION**

(43) Date of publication:
**18.06.2025 Bulletin 2025/25**

(21) Application number: **23216396.4**

(22) Date of filing: **13.12.2023**

(51) International Patent Classification (IPC):
**H10N 60/01** (2023.01)    **H10N 60/12** (2023.01)
**G06N 10/40** (2022.01)

(52) Cooperative Patent Classification (CPC):
**H10N 60/0912; G06N 10/00; G06N 10/40;
H10N 60/12**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Terra Quantum AG
9000 St. Gallen (CH)**

(72) Inventors:
• **Gubaydullin, Azat
9000 St. Gallen (CH)**

• **Perelshtein, Michael R.
9000 St. Gallen (CH)**
• **Vinokour, Valerii
9000 St. Gallen (CH)**
• **Hakonen, Pertti J.
00076 Aalto (FI)**

(74) Representative: **Lucke, Andreas
Boehmert & Boehmert
Anwaltspartnerschaft mbB
Pettenkoferstrasse 22
80336 München (DE)**

(54) **ELEVATED-TEMPERATURE SUPERCONDUCTING QUBITS WITH DISORDER-INDUCED TUNNEL BARRIERS**

(57)    Disclosed herein is a superconducting qubit circuit and a method of forming a superconducting qubit. The superconducting qubit circuit comprises a superconducting qubit, wherein said superconducting qubit comprises a tunnel junction (100) in a superconducting material having a critical temperature above 1.2 K. The tunnel junction is formed by a disorder-induced tunnel barrier (108), wherein said disorder is created by spatial crystallographic defects in said superconducting material.

Fig. 1a

EP 4 572 590 A1

**Description**

FIELD OF THE INVENTION

The present invention is in the field of quantum computing. In particular, the present invention relates to a superconducting qubit circuit with a superconducting qubit comprising a tunnel junction and a method of forming a superconducting qubit comprising a tunnel junction.

BACKGROUND

A quantum computer may solve certain classes of problems more quickly than a classical computer. This is achieved by the use of qubits, i.e., quantum-mechanical two-level systems which may assume a quantum-mechanical superposition of two states ("0" and "1"), instead of classical bits which may only be in either of the two states.

Challenges in the implementation of a quantum computer concern the implementation and manipulation of the qubits. A promising approach uses qubits based on tunnel junctions such as Josephson junctions in (or between) superconductors. Low-temperature superconductors, for example, elemental superconductors such as aluminum (Al), have been used for forming Josephson junctions and qubits with coherence times approaching a millisecond. In a pressing quest of emerging quantum technologies, technology based on Josephson junctions and qubits based on aluminum has successfully demonstrated quantum supremacy and produced a first generation of commercial quantum computers.

However, despite their high quality and merits, the Al Josephson junctions and, in general, all qubit devices based on low-temperature superconductors have some intrinsic limits due to the relatively low quality factor of the Josephson junctions (the quality factor, or characteristic voltage, of a junction may be defined as the product between its critical current and the value of the normal state resistance) as well as due to the low critical temperature and the small superconducting gap of aluminum (namely, about 1.2 K and about 50 GHz / 0.2 meV, respectively), which affect the dynamics of the Al Josephson junction and the qubit device and typically require operating temperatures in the low mK range. In addition, the Al Josephson junction is strongly affected by magnetic fields because of the intrinsic superconducting properties of Al, such as the small superconducting gap. The low operating temperatures in particular cause significant technological challenges and costs in operation of a quantum computer comprising qubits based on a low-temperature superconductor, for example requiring the use of a dilution refrigerator to cool the whole quantum processor and its components, including the qubits and Josephson junctions. In addition to the need for low operating temperatures, read and write frequencies of qubits comprising low-temperature superconductor Josephson junctions may also be limited. The same problems of qubits based on low-temperature superconductors also apply when such qubits are used in other quantum devices, such as a quantum sensor.

While superconducting materials having a higher critical temperature and/or a larger superconducting energy gap than aluminum have been known for a long time, such materials often pose other challenges, for example with regard to the fabrication of Josephson junctions in such materials and in particular with regard to the suitability of such devices for use in qubits.

SUMMARY OF THE INVENTION

The object of the invention is therefore to improve superconducting qubits so as to provide higher operating temperatures, higher read and/or write frequencies, an improved critical current, an improved quality factor, improved noise characteristics, an improved critical magnetic field, and/or a reduced sensitivity to a magnetic field.

This object is met by a superconducting qubit circuit with a superconducting qubit comprising a tunnel junction according to claim 1 and a method of forming a superconducting qubit comprising a tunnel junction according to claim 10. Embodiments of the present invention are detailed in the dependent claims.

According to a first aspect of the present invention, a superconducting qubit circuit comprising a superconducting qubit is provided. The superconducting qubit comprises a tunnel junction in a superconducting material having a critical temperature above 1.2 K. The tunnel junction is formed by a disorder-induced tunnel barrier, wherein the disorder is created by spatial crystallographic defects in the superconducting material.

In the context of the present disclosure, a qubit may denote a quantum-mechanical two-level system, in particular a quantum-mechanical two-level system suitable for quantum information processing. In some embodiments, said quantum-mechanical two-level system may be an effective two-level system, i.e., may comprise, in addition to said two levels (referred to as the "ground state" and "exited state", respectively), one or more additional levels or states, which, however, may be sufficiently far detuned or spaced apart so as to avoid populating such states. A superconducting qubit may denote a qubit that is formed, at least in part, from (e.g., comprises or consists of) a superconducting material (i.e., a material exhibiting a vanishing electrical resistance below a critical temperature), e.g., comprises one or more superconducting structures such as one or more superconducting islands, one or more superconducting electrodes, one or more superconducting contacts and/or one or more superconducting lines or wires.

In the context of the present disclosure, a tunnel junction may for example refer to a junction, connection or link between two (or more) structures, in particular superconducting structures, through which particles and/or quasiparticles, in particular Cooper pairs, may tunnel (e.g., from one of said two or more structures to another one of said two or more structures). Put differently, the tunnel junction may be formed by (e.g., comprise) a tunnel barrier separating said two or more structures. The tunnel junction may thereby form a weak link between said two or more structures. The tunnel junction may in particular be a Josephson junction (JJ), for example a superconductor-insulator-superconductor Josephson junction (S-I-S), a superconductor-normal conductor-superconductor Josephson junction (S-N-S), a Josephson junction formed by a physical constriction (e.g., a point contact) between superconductors (S-c-S) or a combination thereof. The tunnel junction may for example be arranged between (e.g., connect) two superconducting structures such as islands, electrodes, contacts and/or lines, in particular between a superconducting island and a larger superconducting reservoir such as a superconducting structure that is substantially larger than the superconducting island, for example a reference electrode such as a ground electrode/contact (e.g., a ground plane). As used herein, a superconducting island may for example refer to a superconducting structure without a (strong) superconducting link or connection to other superconducting structures. A superconducting island may for example be electrically isolated from other superconducting structures except for, optionally, one or more weak links such as tunnel junctions. The tunnel junction may comprise a physical constriction, e.g., may have a smaller cross-sectional area, preferably a substantially smaller cross-sectional area (e.g., less than 50%, in some examples less than 30%, in one example less than 10% of) than the superconducting structures coupled via the tunnel junction. In some examples, the superconducting qubit circuit, in particular the superconducting qubit may comprise a plurality of tunnel junctions in the superconducting material, for example a pair of tunnel junctions (e.g., as part of a SQUID).

The superconducting qubit (which in short may also be referred to as qubit in the following) comprises (e.g., is formed in and/or of) a superconducting material, in which the tunnel junction is formed. The term "superconducting material", as used herein, may refer to a material that becomes superconducting below a respective critical temperature, i.e., exhibits a vanishing electrical resistance and/or expels magnetic fields below the critical temperature. The qubit comprises a superconducting material having a critical temperature above 1.2 K, i.e., a higher critical temperature than aluminum. Such superconducting materials may also be referred to as "elevated-temperature superconducting materials" or "elevated-temperature superconductors" herein, without, however, implying any limitation (apart from the aforementioned critical temperature) in terms of the material used. In particular, such materials are not limited to cuprate superconductors and/or superconductors having a critical temperature above the boiling point of liquid nitrogen ("high-temperature superconductors").

The superconducting material may have a critical temperature of at least 1.5 K, in some examples of at least 2.0 K, preferably of at least 4.2 K (boiling point of liquid helium), in some examples of at least 5 K, most preferably of at least 10 K, in one example of at least 15 K and in one example of at least 20 K. The superconducting material may for example have a critical temperature of between 4.2 K and 200 K, in some examples of between 4.2 K and 50 K, in one example of between 5 K and 30 K and in one example of between 10 K and 20 K.

The superconducting material may have a superconducting energy gap that is larger than the superconducting energy gap of aluminum (0.2 meV). The superconducting material may for example have a superconducting energy gap of more than 0.3 meV, in some examples of at least 0.5 meV, preferably of at least 1.0 meV, in some examples of at least 1.5 meV, most preferably of at least 2.0 meV, in one example of at least 2.5 meV and in one example of at least 3.0 meV.

The superconducting qubit circuit may be for (i.e., may be suitable or configured for) operation at an operating temperature of at least 1.0 K, preferably of at least 1.5 K, most preferably of at least 2.0 K, in one example of at least 3.0 K and in one example of at least 4.2 K. The stated operating temperatures may for example be achieved by an appropriate choice of one or more of the superconducting material (e.g., a nitride-based superconductor) and its critical temperature, a substrate material (e.g., silicon, sapphire or MgO) and parameters of the qubit and the tunnel junction such as its shape and/or physical dimensions (e.g., the cross-sectional area and/or length of the tunnel junction), the normal state resistance, total capacitance and/or critical current of the tunnel junction, and/or the energy splitting, Josephson energy and/or charging energy of the qubit, e.g., as detailed below. The (maximum) operating temperature of the qubit circuit may for example be limited by thermal excitations of the qubit (e.g., to the excited state or other higher-energy states of the qubit) and/or by quasiparticle excitations in the superconducting material, both of which may disrupt coherence. The energy splitting of the qubit and/or the superconducting energy gap and/or the critical temperature of the superconducting material may for example be chosen such that one or both of the energy splitting and the superconducting energy gap is smaller than, preferably substantially smaller than (e.g., less than 50%, in some examples less than 30%, in one example less than 10% and in one example less than 5% of) the thermal energy $k_B T_O$ associated with the operating temperature $T_O$. Additionally or alternatively, the coherence and/or the maximum operating temperature of the qubit may be improved by reducing the qubit's sensitivity to quasiparticle tunneling across the tunnel barrier and/or to charge fluctuations (for example by an appropriate choice of the ratio $E_J/E_C$ of the Josephson energy $E_J$ and the charging energy $E_C$, e.g., as detailed below), by reducing the qubit's sensitivity to environmental noise (e.g., magnetic field and/or charge noise) and/or by reducing dielectric loss (e.g., by an appropriate combination of superconducting material and substrate material). Improved coherence times may translate into higher maximum temperatures as the qubit can maintain its quantum state for a longer period of time before thermal excitations disrupt the quantum state.

The superconducting material of the qubit may be a single material (e.g., a single metal or alloy) or may be a hybrid or composite material comprising a plurality of different materials (e.g., a combination of different metals and/or alloys), for example a first structure of a first material (e.g., on a first side of the tunnel junction) and a second structure of a second material (e.g., on a second side of the tunnel junction opposite the first side), wherein each of said materials is superconducting with a critical temperature above 1.2 K. The superconducting material of the qubit may be or comprise a type-I superconductor and/or a type-II superconductor. The superconducting material of the qubit may be or comprise an s-wave superconductor and/or a d-wave superconductor.

The superconducting material may for example be or comprise a nitride-based superconductor, such as for example one or more of niobium titanium nitride (NbTiN), niobium nitride (NbN) and titanium nitride (TiN). Additionally or alternatively, the superconducting material may, e.g., be or comprise one or more of yttrium barium copper oxide (YBCO), magnesium diboride (MgB2), bismuth strontium calcium copper oxide (BSCCO) and an iron-based superconductor, in particular an iron-pnictide superconductor. In one example, the superconducting material is or comprises NbTiN, for example $Nb_xTi_{1-x}N$, wherein x may for example be between 0.4 and 0.8, in some examples between 0.5 and 0.7, in one example between 0.6 and 0.65. These materials, for example, have critical temperatures and/or superconducting energy gaps within the aforementioned ranges.

The tunnel junction (which in short may also be referred to as junction in the following) of the qubit is formed by a disorder-induced tunnel barrier in the superconducting material. As used herein, the term "disorder" may for example refer to random or substantially random deviations within the superconducting material from its ideal structure and/or composition (e.g., from its ideal crystal structure in the absence of any impurities). While weak disorder typically does not affect superconductivity, stronger disorder may cause a breakdown of the superconducting state and may, e.g., lead to a superconductor-to-metal or superconductor-to-insulator transition. This may for example occur when the disorder is so strong that the mean free path becomes comparable to the Fermi wavelength (e.g., of the same order of magnitude). A disordered region may thus create a tunnel barrier within the superconducting material, thereby forming the tunnel junction.

The disorder is created by spatial crystallographic defects in the superconducting material. In the context of the present disclosure, a "spatial crystallographic defect" may for example refer to a spatial deviation, dislocation or interruption within the crystal structure of the superconducting material, e.g., a lattice position or site in the ideal crystal structure of the superconducting material not being occupied by an atom (e.g., a vacancy defect) and/or an atom being arranged at a position different from (e.g., being displaced from) the lattice positions in the ideal crystal structure of the superconducting material (e.g., an interstitial defect). As such, spatial crystallographic defects are to be distinguished from impurity-based disorder or crystallographic defects such as substitutional defects, wherein an impurity atom (which otherwise is not supposed to be in the superconducting material) occupies a regular lattice position in the ideal crystal structure (for example as a result of ion implantation as, e.g., used in US 11,538,977 B2). The spatial crystallographic defects may thus also be referred to as "non-impurity crystallographic defects" or "dislocations" herein. While there may (and typically will) also be impurities present in the superconducting material to at least some extent, the spatial crystallographic defects are the dominant form of disorder in the tunnel barrier of the superconducting qubit according to the present invention. The spatial crystallographic defects may in particular be the form of disorder that ultimately is responsible for the breakdown of superconductivity in the tunnel barrier, which may thus also be referred to as a "spatial crystallographic defect-induced tunnel barrier" or "dislocation-induced tunnel barrier" in short. Put differently, the amount of impurities present in the tunnel barrier may be below, preferably substantially below the threshold required for the superconductivity to break down. For example, a mean free path between impurities may be at least an order of magnitude smaller than (e.g., less than 10% of), preferably at least two orders of magnitude smaller than (e.g., less than 1% of) the Fermi wavelength in the superconducting material. In some examples, a ratio between an amount (e.g., a number or a density) of impurities (e.g., substitutional defects) and an amount of non-impurity crystallographic defects may be less than 0.5, preferably less than 0.2, most preferably less than 0.1, in some examples less than 0.05, in one example less than 0.02 and in one example less than 0.01. The spatial crystallographic defects may be or comprise point defects (e.g., at a single lattice position, for example a single vacancy defect, a single interstitial defect or a Frenkel defect or pair), line defects (e.g., an edge dislocation or a screw dislocation), planar defects or combinations thereof. Disorder-induced tunnel barriers may offer the advantages of having no interfaces between different materials (as, e.g., in Al/AlO/Al junctions) and/or allowing for placing the tunnel barrier/junctions much closer to each other. This may for example be particularly advantageous for fluxonium qubits and for exhibiting flux-focusing effects in magnetic field measurements.

In a preferred embodiment, the spatial crystallographic defects in the tunnel barrier were created by irradiation with ions, in particular silicon ions and/or noble gas ions such as helium ions and/or neon ions, for example using a method according to the second aspect of the present invention described below. The spatial crystallographic defects in the tunnel barrier may in particular have been created by irradiation with a focused ion beam, for example a focused ion beam of noble gas ions and/or silicon ions, e.g., as detailed below. For a given thickness (e.g., film thickness) of the superconducting material, an energy of the ions may be chosen such that the ions penetrate through the superconducting material (e.g., into an underlying substrate), preferably with no or minimal lateral spreading (e.g., without substantial increase in the diameter of the focused ion beam). Kinetic energy deposited into the superconducting material by the ions may result in the creation of defects, e.g., through local displacement of atoms. Without wishing to limit the present invention to any particular mechanism for creating spatial crystallographic defects and without being bound to any particular theory, disorder may for example by introduced by one or both of the following two mechanisms: 1) ions may remove (e.g., knock out) atoms from the lattice, thereby creating vacancies and/or surface roughness (which may, e.g., be visible in cross-sectional images) and 2) ions may displace atoms, causing them to move from their original or intrinsic positions in the lattice with the displaced atoms (which may not be removed from the superconducting material entirely) becoming defects within the lattice (which may also include the vacancy created thereby). As the ions penetrate (and thus do not remain within) the superconducting material, the irradiation may not (or only to a small extent) create impurities in the superconducting material. There may, however, already be a few impurities present in the superconducting material prior to the irradiation (e.g., as a result of the deposition of the superconducting material), some of which may also be displaced by the ions. The high ionization potential of the ions (e.g., of helium and/or neon ions) and/or their high kinetic energy may also result in most impurity atoms or contaminants, e.g., on the sample surface and/or in the apparatus used for the irradiation (e.g., the focused-ion beam system and/or ion microscope) being ionized early in the process and not forming a significant part of the ions (e.g., the ion beam) irradiating (e.g., penetrating through) the sample.

Preferably, the tunnel barrier is free of aluminum oxide. In other words, the tunnel barrier (and in some examples the entire tunnel junction, e.g., also including portions of the superconducting material adjacent to the tunnel barrier) may not contain aluminum oxide in stoichiometrically relevant quantities. For example, the aluminum oxide content in the tunnel barrier and/or in the tunnel junction may be less than $10^{-3}$, preferably less than $10^{-4}$, most preferably less than $10^{-5}$. In some examples, the tunnel barrier and/or the tunnel junction may be free of aluminum (i.e., aluminum in any form including elemental aluminum). For example, the aluminum content in the tunnel barrier and/or in the tunnel junction may be less than $10^{-3}$, preferably less than $10^{-4}$, most preferably less than $10^{-5}$.

A cross-sectional area of the tunnel barrier may be less than $3.0 \cdot 10^4$ nm$^2$, preferably less than $2.0 \cdot 10^4$ nm$^2$, most preferably less than $1.5 \cdot 10^4$ nm$^2$, in some examples less than less than $1.0 \cdot 10^4$ nm$^2$, in some examples less than $5 \cdot 10^3$ nm$^2$, in some examples less than $3 \cdot 10^3$ nm$^2$, in some examples less than $2 \cdot 10^3$ nm$^2$, in some examples less than $1 \cdot 10^3$ nm$^2$ and in one example less than $5 \cdot 10^2$ nm$^2$. The cross-sectional area of the tunnel barrier may be measured laterally across the tunnel barrier, for example in a plane perpendicular to a direction of current flow through the tunnel barrier. The cross-sectional area may for example be an important parameter affecting quantities such as the critical current, the normal state resistance and/or the capacitance of the tunnel junction, which may determine the quality of the tunnel junction and of the superconducting qubit as a whole, in particular their suitability for quantum information processing.

A length ("width") of the tunnel barrier may be between 0.5 nm and 50 nm, preferably between 1 nm and 25 nm, in some examples between 2 nm and 20 nm, most preferably between 5 nm and 15 nm. Additionally or alternatively, the length of the tunnel barrier may for example be on the same order of magnitude as (e.g., between 0.2 times and 20 times, in some examples between 0.5 times and 10 times, in one example between 2 times and 6 times of) one or both of the superconducting coherence length and the penetration depth of the superconducting material. The length of the tunnel barrier may be measured longitudinally across the tunnel barrier, for example parallel to a direction of current flow through the tunnel barrier. Such short/thin tunnel barriers may for example be created by irradiation with a focused ion beam, e.g., as described below. Using such short/thin tunnel barriers may allow for strong tunnel coupling across the tunnel junction, even for superconducting material having short coherence lengths such as NbTiN (about 2.5 nm) or NbN (about 5 nm), which in turn may be advantageous in other aspects such as for example improved energy confinement and/or reduced energy loss within the superconducting qubit circuit, in particular a readout circuit thereof.

A normal state resistance $R_N$ of the tunnel junction may be at least 100 Ω, preferably at least 200 Ω, most preferably at least 300 Ω, in some examples at least 400 Ω and in one example at least 500 Ω. The normal state resistance of the tunnel junction may for example be between 100 Ω and 2 kΩ, in some examples between 200 Ω and 1 kΩ, in one example between 300 Ω and 500 Ω. Additionally or alternatively, a total capacitance of the superconducting qubit (e.g., of a superconducting island thereof coupled via the tunnel junction) is at least 10 fF, preferably at least 20 fF, most preferably at least 50 fF. The total capacitance of the superconducting qubit may for example be between 10 fF and 1000 fF, in some examples between 20 fF and 500 fF, in one example between 40 fF and 200 fF and in one example between 50 fF and 100 fF. The total capacitance $C_\Sigma$ of the superconducting qubit (which may also be referred to as its self-capacitance) may for example comprise an intrinsic capacitance of the tunnel junction $C_J$ and, optionally, one or more additional capacitances in parallel to the tunnel junction, in particular an additional shunt capacitance $C_o$ (for example to ground) and/or a gate capacitance $C_g$ of the superconducting qubit, e.g., $C_\Sigma = C_J + C_o + C_g$. The normal state resistance of the tunnel junction and the total capacitance of the superconducting qubit may for example determine properties of the superconducting qubit such as the energy splitting, its anharmonicity and/or its sensitivity to charge noise, e.g., as detailed below.

Additionally or alternatively, a critical current of the tunnel junction may be less than 1.0 μA, preferably less than

0.5 $\mu$A, in some examples less than 0.3 $\mu$A, most preferably less than 0.2 $\mu$A, in one example less than 0.1 $\mu$A and in one example less than 0.05 $\mu$A. The critical current of the tunnel junction may for example be between 1 nA and 1.0 $\mu$A, in some examples between 5 nA and 0.5 $\mu$A, in one example between 10 nA and 0.1 $\mu$A. While resulting in a lower quality factor/characteristic voltage of the tunnel junction, a low critical current may be advantageous for retaining a suitable degree of anharmonicity of the qubit (to obtain a well-isolated effective two-level system) while also suppressing charge noise, e.g., as detailed below, and may allow for implementing high-frequency qubits with large energy splittings (e.g., of at least 10 GHz, preferably of at least 20 GHz). To nonetheless achieve a desired quality factor, the normal state resistance may be adjusted (e.g., increased) accordingly. Previously known isolated/single disorder-based tunnel junctions as for example described in A. Ruhtinas and I. J. Maasilta, arXiv:2303.17348v1 [cond-mat.supr-*con]* had substantially larger critical currents (and cross-sectional areas as well as smaller normal state resistances), rendering them unsuitable for use in superconducting qubits, in particular high-frequency superconducting qubits.

In a preferred embodiment, the superconducting qubit is a charge qubit, e.g., a Cooper pair box. The basis sates of the superconducting qubit may for example be charge states of a superconducting island coupled via the tunnel junction (e.g., to a larger reservoir such as a larger superconducting contact, electrode or line, in particular a ground contact or electrode). The superconducting qubit may in particular be a SQUID-based charge qubit, i.e., a charge qubit comprising a superconducting quantum interference device (SQUID) formed by a two or more tunnel junctions in a superconducting loop. SQUID-based charge qubits may for example allow for flux tuning of the qubit by adjusting a magnetic flux through the SQUID (e.g., a flux threading the superconducting loop), for example to tune a Josephson energy of the tunnel junctions. In other examples, the superconducting qubit may also be a different type of qubit such as for example a phase qubit, a flux qubit or a combination of two or more of a charge qubit, a phase qubit and a flux qubit.

Preferably, a ratio $E_J/E_C$ of a Josephson energy $E_J$ and a charging energy $E_C$ of the superconducting qubit (which may, e.g., be a charge qubit as mentioned above) is between 20 and 2 000, in some examples between 50 and 1 000, in some examples between 100 and 1 000, preferably between 200 and 600, in one example between 300 and 500. The Josephson energy may for example be the energy associated with tunnel processes (e.g., of a Cooper pair) across the tunnel junction, which may be proportional to the critical current $I_C$ of the tunnel junction, $E_J = \Phi_0 I_C/(2\pi)$ with $\Phi_0$ denoting the magnetic flux quantum, and may determine the energy stored in the tunnel junction when a current passes therethrough. The charging energy $E_C$ may for example be the energy associated with charging the superconducting qubit (e.g., the superconducting island coupled via the tunnel junction), for example by adding a Cooper pair. The charging energy $E_C$ may depend on the total capacitance $C_\Sigma$ of the qubit and may, e.g., be given by $E_C = e^2/(2C_\Sigma)$ with e denoting the elementary charge. A ratio $E_J/E_C$ within the aforementioned ranges may for example allow for reducing the sensitivity of the qubit to charge noise while retaining a sufficient degree of anharmonicity. In some examples, the qubit may be embodied as a transmon qubit (e.g., a charge qubit comprising the tunnel junction and an additional shunt capacitance in parallel, for example as described in J. Koch et al., Phys. Rev. A 76, 042319 (2007*)* or a variation thereof). The qubit may in particular be embodied as an Xmon qubit (e.g., as described in R. Barends et al., Phys. Rev. Lett. 111, 080502 (2013*)* or a variation thereof). In other examples the qubit may for example be a Gatemon qubit.

An energy splitting of the superconducting qubit may for example be between 1 GHz and 500 GHz, in some examples between 5 GHz and 200 GHz, preferably between 10 GHz and 100 GHz, most preferably between 20 GHz and 50 GHz. The energy splitting, as used herein, may for example refer to the energy difference between the ground state and the excited state forming the two-level (or effective two-level) system of the superconducting qubit. Such large energy splitting may, e.g., be enabled by the large superconducting energy gap of the superconducting material and may, e.g., allow for high operating frequencies (e.g., read and/or write frequencies).

In some embodiments, the superconducting qubit circuit may comprise one or more additional elements in addition to the superconducting qubit, whereas in other examples the superconducting qubit circuit as claimed may only consist of the superconducting qubit. Some or all of the elements of the superconducting qubit circuit may be formed of (e.g., comprise or consist of) a superconducting material, preferably of the same superconducting material as the superconducting qubit. In addition, the superconducting qubit circuit may also comprise one or more normal conducting elements and/or structures and/or one or more insulating elements and/or structures.

The superconducting qubit circuit may for example comprise one or more of a readout electrode and/or a readout circuit (which may comprise and/or be coupled to the readout electrode) for reading out a state of the superconducting qubit, a control electrode for manipulating a state of the superconducting qubit and a flux bias line for adjusting the energy splitting of said superconducting qubit. The control line may be comprised in and/or coupled to a control circuit for manipulating the state of the superconducting qubit, wherein the control circuit may also be part of the superconducting qubit circuit in some examples or alternatively may be provided as a separate unit. The flux bias line may be comprised in and/or coupled to a flux bias circuit for adjusting the energy splitting of said superconducting qubit, wherein the flux bias circuit may also be part of the superconducting qubit circuit in some examples or alternatively may be provided as a separate unit. The readout electrode and/or the control electrode may be coupled (e.g., capacitively) to the superconducting qubit, in particular to the superconducting island thereof. The flux bias line may be configured to generate a magnetic field at the superconducting qubit (e.g., when a current is applied to the flux bias line), in particular a magnetic field threading the SQUID loop of the qubit. For this, the flux bias line may, e.g., be coupled inductively to the superconducting qubit.

In some examples, the superconducting material of the qubit may be a superconducting film (e.g., formed or deposited as a superconducting film or layer). The superconducting material may for example be a superconducting film having a thickness of less than 0.5 μm, preferably of less than 0.2 μm, in some examples of less than 100 nm, in one example of less than 50 nm and in one example of less than 25 nm. The superconducting film may be arranged on a substrate, in particular an insulating substrate. The superconducting film may comprise one or more layers. The superconducting material may in particular be a superconducting thin film. The term "thin film", as used herein, may for example refer to films having a thickness that is sufficiently low such that one or more properties pertaining to superconductivity within the film such as for example the critical temperature differ from the respective properties of the bulk material. In such cases, the critical temperature of the superconducting material as used herein may refer to the critical temperature of the film (rather than the one of the bulk material).

The tunnel junction and one or more of, preferably all of the readout line, the readout circuit, the control line and the flux bias line (as well as, optionally, the control circuit and/or the flux bias circuit) are made of (e.g., comprise and/or consist of) the same superconducting material (i.e., the elevated-temperature superconducting material of the qubit in which the tunnel junction is formed). The tunnel junction and said one or more of, preferably all of the readout line, the readout circuit, the control line and the flux bias line (as well as, optionally, the control circuit and/or the flux bias circuit) may in particular be made of a same film of the superconducting material, for example by patterning the film of the superconducting material to form the respective circuit elements.

In some examples, the superconducting qubit circuit may comprise a plurality of qubits, for example (but not limited to) at least 2 qubits, in some examples at least 4 qubits, in some examples at least 8 qubits, in some examples at least 16 qubits, in one example at least 64 qubits and in one example at least 128 qubits. The present invention is, however, not limited to any particular number of qubits and the superconducting qubit circuit may comprise any number of qubits. Each of said qubits may for example be embodied as described above. Additionally or alternatively, the superconducting qubit circuit may comprise one or more quantum gates (quantum logic gates), each of which may act on one or more qubits, preferably on two or more qubits. In some examples, the superconducting qubit circuit may be or comprise a quantum processor or may be for use in (and in some examples part of) a quantum processor. Additionally or alternatively, the superconducting qubit circuit may be or comprise a quantum sensor or may be for use in (and in some examples part of) a quantum sensor. In some examples, the superconducting qubit circuit may be embodied as or arranged on and/or in a chip or a chip package.

According to a second aspect of the present invention, a method of forming a superconducting qubit comprising a tunnel junction is provided. The method comprises forming a superconducting structure of the superconducting qubit, the superconducting structure being formed of a superconducting material having a critical temperature above 1.2 K. The method further comprises forming the tunnel junction by creating a disorder-induced tunnel barrier in the superconducting structure, wherein the tunnel barrier is created by introducing spatial crystallographic defects into the superconducting material.

The superconducting qubit, the tunnel junction, the superconducting material, the tunnel barrier and/or the spatial crystallographic defects may for example be embodied and/or formed as in the superconducting qubit circuit according to the first aspect of the present invention in accordance with any one of the embodiments described herein, i.e., may have or comprise some or all of the features of the respective element of the superconducting qubit circuit according to the first aspect as disclosed herein. The method according to the second aspect may be used for forming the superconducting qubit circuit according to the first aspect or a part thereof, in particular the superconducting qubit thereof. The execution of the method is not limited to a particular order. As far as technically feasible, the method may be executed in an arbitrary order and steps thereof may also be executed simultaneously at least in part. For example, the spatial crystallographic defects may be introduced into the superconducting material prior to, while and/or after forming the superconducting structure.

The superconducting structure of the qubit may for example be or comprise one or more superconducting islands and/or one or more superconducting reservoirs, e.g., as described above. The tunnel junction may be formed so as to couple the superconducting island(s) to the superconducting reservoir(s), e.g., to provide a weak link therebetween. In some examples, a plurality of tunnel junctions may be formed, for example a pair of tunnel junctions (e.g., for forming a SQUID). Additionally or alternatively, the superconducting structure may be or comprise one or more other structures such as one or more superconducting electrodes, one or more superconducting contacts and/or one or more superconducting lines or wires. The superconducting structure may consist of (e.g., be formed exclusively of) or comprise (e.g., be formed of, among others) the elevated-temperature superconducting material.

For forming the tunnel junction, a disorder-induced tunnel barrier is created in the superconducting structure, wherein the tunnel barrier may be created prior to, during and/or after forming the superconducting structure. The tunnel barrier is created by introducing spatial crystallographic defects into the superconducting material, i.e., is a "spatial crystallographic defect-induced tunnel barrier" or "dislocation-induced tunnel barrier" as described above. This may comprise introducing such defects to an extent (e.g., in a number and/or density) so as to cause breakdown of superconductivity in the superconducting material, e.g., to cause a superconductor-to-insulator transition or a superconductor-to-normal-conductor transition (e.g., superconductor-to-metal transition). In some examples, forming the tunnel junction may also comprise forming a physical constriction, e.g., within or bordering the superconducting structure of the qubit. The tunnel barrier may be created in said physical constriction.

The spatial crystallographic defects in the superconducting material may for example be introduced by irradiation of the superconducting material, in particular high-energy irradiation (e.g., with particles having an energy of at least 1 keV, preferably of at least 5 keV, in some examples of at least 10 keV, in one example of at least 20 keV, in one example of at least 30 keV and in one example of at least 40 keV). The particle energy may for example be chosen based on a thickness (e.g., a film thickness) of the superconducting material and/or a length of the tunnel barrier. For example, for a superconducting material of larger thickness (e.g., for tuning the kinetic inductance and/or increasing the critical temperature), a higher particle energy may be used that for a superconducting material of smaller thickness. The irradiation may be or comprise electromagnetic irradiation (e.g., by X-ray and/or gamma radiation) and/or irradiation by massive particles such as electrons and/or ions, e.g., with a focused electron beam and/or a focused ion beam. The irradiation may be a targeted irradiation, e.g., using a focused beam. Preferably, the irradiation is mask-free, i.e., the superconducting material is irradiated without a mask for blocking the radiation being arranged thereon. Additionally or alternatively, the spatial crystallographic defects may also be introduced when forming (e.g., depositing, growing and/or patterning) the superconducting material.

The irradiation for introducing the spatial crystallographic defects may be adapted to the superconducting material used and/or to the superconducting structure, in particular to one or more physical dimensions thereof, for example a length of the tunnel barrier and/or a thickness of the superconducting structure/material (e.g., a film thickness). For this, parameters of the irradiation such as a type of radiation (e.g., electromagnetic vs. ion radiation), a species of the particles of the radiation (e.g., the species of ions used), a particle energy (e.g., an acceleration voltage and/or a wavelength), a dose, a fluence (e.g., an intensity and/or an ion beam current) and/or a dwell time may be adapted to (e.g., chosen based on) the superconducting material used and/or the superconducting structure. Preferably, the irradiation is adapted so as to allow the irradiation to penetrate through the superconducting material and/or the superconducting structure, e.g., into a substrate below the superconducting material and the superconducting structure, respectively, preferably with no or minimal lateral spreading.

In a preferred embodiment, the spatial crystallographic defects in the superconducting material are introduced by irradiating the superconducting material with noble gas ions, in particular helium ions. Additionally or alternatively, silicon ions and/or other noble gas ions such as, e.g., neon ions may also be used.

The spatial crystallographic defects in the superconducting material may be introduced by irradiation with a focused ion beam, in particular with a focused ion beam of noble gas ions and/or silicon ions, for example a focused helium ion beam and/or a focused neon ion beam. Using a focused ion beam may facilitate a targeted and well-controlled introduction of spatial crystallographic defects in certain portions or areas of the superconducting material or structure even at very small length scales, thus allowing for "direct writing" of the disorder-induced tunnel barrier in the superconducting structure. A spot size of the focused ion beam at the superconducting material may for example be below 20 nm, in some examples below 10 nm, preferably below 5 nm, most preferably below 2 nm, in one example below 1 nm and in one example below 500 pm. The spot size may for example be between 100 pm and 5 nm, in some examples between 200 pm and 2 nm and in some examples between 400 pm and 1.2 nm.

The superconducting material may for example be irradiated with ions, in particular noble gas ions such as helium and/or neon ions and/or silicon ions, with a dose of between $10^{16}$ ions/cm$^2$ and $10^{22}$ ions/cm$^2$, preferably of between $10^{17}$ ions/cm$^2$ and $10^{21}$ ions/cm$^2$, most preferably of between $10^{18}$ ions/cm$^2$ and $10^{20}$ ions/cm$^2$ and in one example of between $5 \cdot 10^{18}$ ions/cm$^2$ and $5 \cdot 10^{19}$ ions/cm$^2$. The dose may for example be chosen based on an amount of disorder, in particular an amount of spatial crystallographic defects to be introduced into the superconducting material, based on a thickness of the superconducting material (e.g., a height of the tunnel barrier) and/or based on the superconducting material used (nitride-based superconductors, for example, may require significantly larger doses (e.g., two orders of magnitude larger doses) to suppress superconductivity than, e.g., YBCO). This may enable precise control of the properties, in particular the electric properties of the tunnel barrier, for example the resistivity of the tunnel barrier (e.g., to form an insulating or metallic tunnel barrier).

The superconducting material may be arranged on a substrate, in particular an insulating substrate. The superconducting material may be irradiated through a surface thereof exposed from (e.g., facing away from) the substrate, e.g., in a direction perpendicular to the surface. A kinetic energy of the ions (e.g., the noble gas ions) may be chosen such that the ions penetrate through the superconducting material into the substrate, for example such that at least 80%, preferably at least 90%, most preferably at least 95%, in some examples at least 98%, in one example at least 99%, in one example at least 99.9% and in one example at least 99.99% of the ions penetrate through the superconducting material into the substrate. This may allow for (primarily) introducing spatial crystallographic defects rather than impurities (in contrast to, e.g., ion implantation into the superconducting material). The kinetic energy of the ions may for example be at least 5 keV, in some examples of at least 10 keV, in one example of at least 20 keV and in one example of at least 30 keV. Preferably, the ions penetrate through the superconducting material with no or minimal lateral spreading, e.g., such that a lateral extent (e.g., a width or diameter) of an irradiated region or spot (e.g., a lateral extent of the focused ion beam) does not change substantially over a thickness of the superconducting material, for example changes (e.g., increases) by less than a factor of two, preferably by less than a factor of 1.5, in one example by less than a factor of 1.2 over the thickness of the superconducting material. This may ensure a uniform disordered region throughout the superconducting material, preserve superconducting properties of other portions of the superconducting material, enable achieving low kinetic inductance and/or allow for reproducibly fabricating tunnel junctions with the desired parameters such as a desired junction length, a desired critical current and/or a desired normal state resistance.

The technique(s) used for forming the superconducting structure is/are not particularly limited and may be or comprise any technique(s) for forming superconducting structures known in the art. Forming the superconducting structure may comprise depositing the superconducting material and/or patterning the superconducting material. The superconducting material may be deposited using one or more depositions techniques known in the art such as, for example, physical vapor deposition (PVD, e.g., sputtering, pulsed laser deposition (PLD) and/or electron beam evaporation), chemical vapor deposition (CVD, for example vapor-liquid-solid growth, e.g., for forming one-dimensional structures such as nanowires), atomic layer deposition (ALD), epitaxial growth (e.g., molecular beam epitaxy, MBE), spin coating and combinations thereof (e.g., hybrid methods combining two or more of the aforementioned deposition techniques, for example a combination of PLD and MBE). In some examples, the superconducting material may be deposited in a patterned fashion, e.g., using a suitable mask and/or targeted deposition or growth. Additionally or alternatively, the superconducting material may be patterned using one or more patterning techniques known in the art such as, for example lithography (e.g., electron lithography and/or photolithography), etching (e.g., wet etching, dry etching and/or reactive ion etching) and/or milling (e.g., ion milling).

In some examples, forming the superconducting structure comprises forming (e.g., depositing) a film of the superconducting material, in particular a thin film. The film may comprise one or more layers. The film of the superconducting material may for example be formed with a thickness as described above for the superconducting qubit circuit according to the first aspect. The thickness of the film may be homogeneous, i.e., the film may be formed as a layer of uniform thickness. Forming the superconducting structure may additionally comprise patterning the film to form the superconducting structure, e.g., using one or more of the aforementioned patterning techniques.

The method may further comprise forming one or more additional structures, e.g., one or more additional structures of a superconducting qubit circuit, for example as detailed above for the superconducting qubit circuit according to the first aspect. The method may for example comprise forming one or more of, preferably all of a readout electrode and/or a readout circuit for reading out a state of the superconducting qubit, a control electrode for manipulating a state of the superconducting qubit and a flux bias line for adjusting an energy splitting of the superconducting qubit. In some examples, the method may also comprise forming a control circuit for manipulating the state of the superconducting qubit, which may comprise and/or be coupled to the control electrode, and/or forming a flux bias circuit for adjusting the energy splitting of said superconducting qubit, which may comprise and/or be coupled to the flux bias line.

One or more of, preferably all of the readout electrode, the readout circuit, the control electrode and the flux bias line (as well as, optionally the control circuit and/or the flux bias circuit) may be formed from the same superconducting material, in particular the same film of the superconducting material as the superconducting structure of the superconducting qubit. In particular, the film may be patterned so as to form the superconducting structure and one or more of, preferably all of the aforementioned structures simultaneously, e.g., in a single patterning step or process (for example using a common mask and/or a common etching step for all of the respective structures).

The superconducting qubit of the superconducting qubit circuit according to the first aspect of the present invention, in some examples the entire superconducting qubit circuit according to the first aspect may be obtainable (e.g., obtained or formed, or obtained or formed so as to have the same structural properties and/or features as if obtained or formed) using a method according to the second aspect of the present invention in accordance with any one of the embodiments disclosed herein.

According to a third aspect, the present invention relates to use of (e.g., a method or process using) the superconducting qubit circuit according to the first aspect in accordance with any one of the embodiments disclosed herein, wherein the superconducting qubit circuit is operated at an operating temperature of at least 1.0 K, preferably of at least 1.5 K, most preferably of at least 2.0 K, in one example of at least 3.0 K and in one example of at least 4.2 K. The superconducting qubit circuit may for example be used for quantum information processing (e.g., in a quantum processor and/or a quantum computer) and/or quantum sensing (e.g., in a quantum sensor).

The superconducting qubit circuit according to the first aspect and the method according the second aspect of the present invention are not limited to elevated-temperature superconducting materials having a critical temperature above 1.2 K but may also be implemented with any other superconducting material, in particular superconducting materials having a critical temperature of 1.2 K or less. Furthermore, the superconducting qubit circuit according to the first aspect and the method according the second aspect of the present invention are not limited to disorder created by spatial crystallographic defects but additionally or alternatively may also be implemented with other types of disorder (i.e., any type of disorder-induced tunnel barrier), in particular disorder created by impurity-based crystallographic defects.

LIST OF FIGURES

In the following, a detailed description of the invention and exemplary embodiments thereof is given with reference to the figures. The figures show schematic illustrations of

Figs. 1a, 1b: a superconducting qubit circuit according to an example in top view and side-view, respectively;

Fig. 2: a flow diagram of a method of forming a superconducting qubit comprising a tunnel junction in accordance with an example;

Fig. 3a: a superconducting qubit circuit with a SQUID-based charge qubit according to an example in top view;

Fig. 3b: the SQUID-based charge qubit of the superconducting qubit circuit of Fig. 3a in a close-up top view;

Fig. 4a: a circuit diagram of a superconducting qubit circuit with a SQUID-based charge qubit, a readout circuit and a control circuit according to an example;

Fig. 4b: a circuit diagram of a flux bias circuit for the superconducting qubit circuit of Fig. 4a; and

Fig. 5: a flow diagram of a method of forming a superconducting qubit circuit with a superconducting qubit comprising a disorder-induced tunnel barrier in accordance with an example.

DESCRIPTION OF THE EXAMPLES

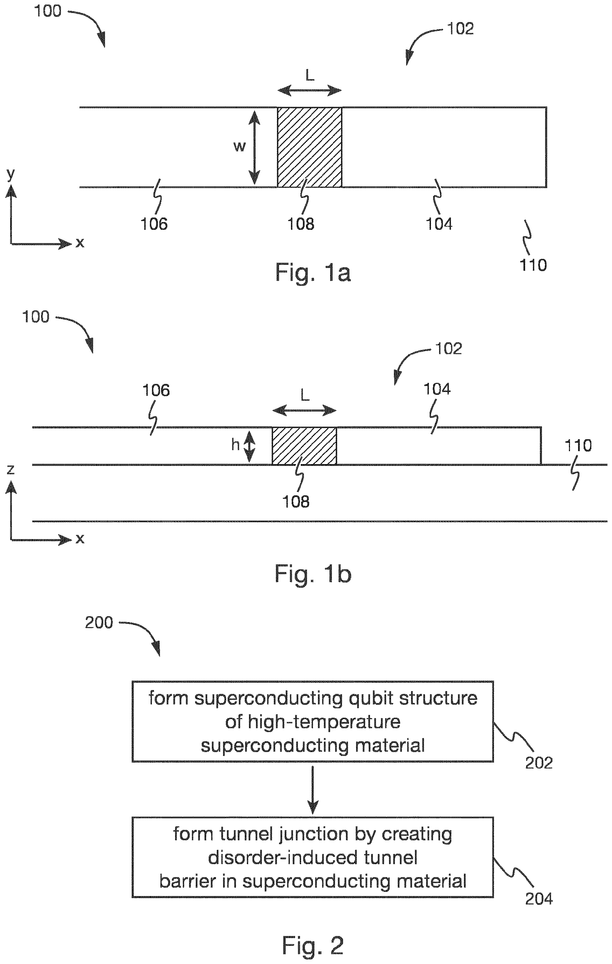

Figs. 1a and 1b depict schematic illustrations of a superconducting qubit circuit 100 according to the first aspect of the present invention in accordance with an example in top view and side view, respectively. The superconducting qubit circuit 100 (referred to in short as the qubit circuit 100 in the following) comprises a superconducting qubit 102, which in this example is formed by a superconducting island 104 that is coupled to a superconducting reservoir 106 (e.g., a larger superconducting electrode or a superconducting line or wire) via a tunnel junction. The tunnel junction, in this example a Josephson junction, is formed by a disorder-induced tunnel barrier 108 arranged between the island 104 and the reservoir 106. The superconducting qubit 102 (referred to in short as the qubit 102 in the following), i.e., the island 104, the reservoir 106 and the tunnel barrier 108, is arranged above (e.g., on) an insulating substrate 110 and formed of (e.g., comprises and/or consists of) an elevated-temperature superconducting material with a critical temperature above the critical temperature of aluminum (1.2 K). The elevated-temperature superconducting material may for example be or comprise NbTiN (Tc = 15 K). In other examples, other nitride-based superconductors such as NbN and/or TiN may for example be used instead of (or in addition to) NbTiN. Preferably, the elevated-temperature superconducting material also has a superconducting energy gap higher than the one of aluminum (0.2 meV) with the superconducting gap of NbTiN, for example, being about 2.0 meV. In some examples, the superconducting energy gap may be proportional or approximately proportional to the critical temperature. The substrate 110 may for example comprise or consist of silicon, sapphire and/or MgO. In some examples, a reference electrode (not shown) such as a ground plate may be arranged on a bottom surface of the substrate 110 opposite the top surface on which the qubit 102 is arranged. The tunnel barrier 108 and preferably the entire qubit 102 may be free of aluminum, i.e., may not contain any stoichiometrically relevant quantities of elemental aluminum or aluminum compounds such as aluminum oxide.

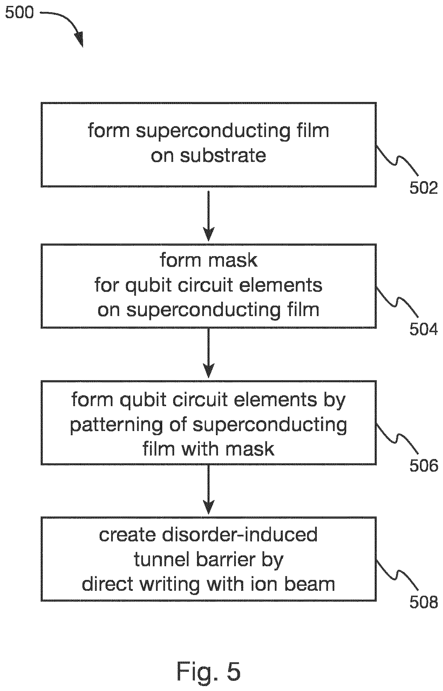

The disorder in the disorder-induced tunnel barrier 108 is created by spatial crystallographic defects in the superconducting material, i.e., by spatial deviations, dislocations and/or interruptions within the crystal structure of the superconducting material. These defects may for example comprise vacancy defects (unoccupied lattice sites) and/or interstitial defects (e.g., atoms displaced from lattice sites). The density of the spatial crystallographic defects is such that the tunnel barrier 108 is no longer in the superconducting state but, e.g., in a normal or insulating state, thereby forming weak link between the island 104 and the reservoir 106. The disorder in the tunnel barrier 108 may, e.g., be created by irradiation with ions, in particular noble gas ions such as helium ions, for example by irradiation with a focused ion beam. The disorder in the tunnel barrier 108 may for example be created as detailed for method 200 of Fig. 2 and/or method 500 of Fig. 5 below.

The qubit 102 may for example be described, at least approximately, by a Hamiltonian of the form (see for example J. Koch et al., Phys. Rev. A 76, 042319 (2007))

$$H = 4E_C\left(\hat{n} - n_g\right)^2 - E_J(\cos\phi)$$

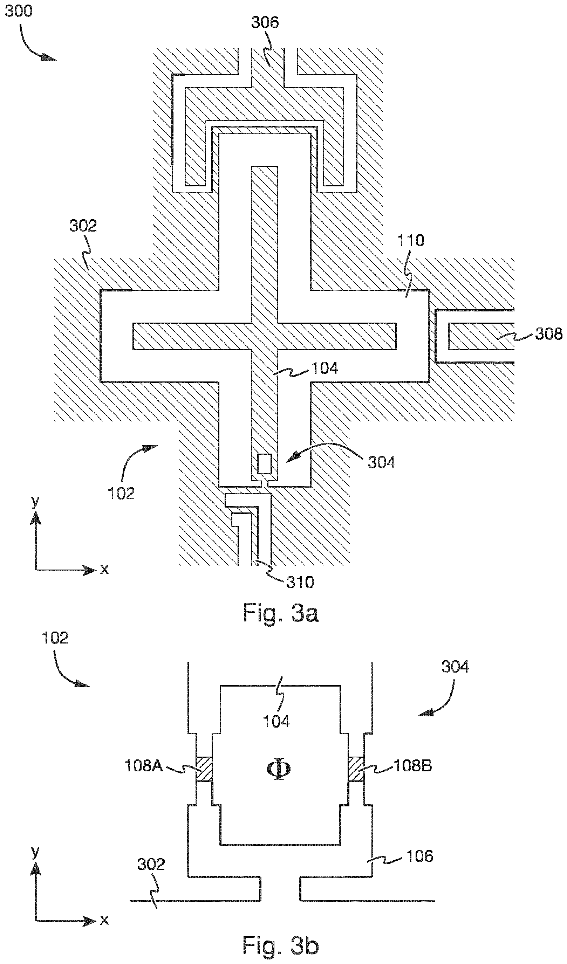

with $E_J = \Phi_0 I_c/(2\pi) = R_Q \Delta/(2\,R_N)$ denoting the Josephson energy of the tunnel junction having a critical current $I_c$, a normal state resistance $R_N$ and a superconducting energy gap $\Delta$ (with $\Phi_0$ and $R_Q$ being the magnetic flux quantum and quantum of resistance, respectively, and $\phi$ the phase difference across the junction) and with $E_C = e^2/2C_\Sigma$ denoting the charging energy of the island 104 having a total capacitance of $C_\Sigma$. The total capacitance of the island 104 may for example be the sum of the capacitance of the tunnel junction $C_J$ and any additional capacitance arranged in parallel thereto such as for example a gate capacitance $C_g$ of a gate (not shown) of the qubit 102 (e.g., for controlling the chemical potential of the island 104) and/or an additional shunt capacitance $C_0$ (which may also be denoted as $C_q$ or $C_{ground}$), which may for example be formed between the island 104 and a reference electrode such as a ground electrode or plane (e.g., as described below with reference to Fig. 3). The number of Cooper pairs on the island 104 is denoted by $\hat{n}$ and $n_g$ is the effective offset charge number.

The energy spacing between the ground state and the first excited state of the qubit 102 may, at least approximately, scale as

$$E_{01} = E_C\left(2\sqrt{2r} - 1\right)$$

with $r = E_J/E_C$ denoting the ratio between the Josephson and charging energies. A smaller ratio r may be associated with a larger anharmonicity of the level structure of the qubit 102 such that the ground and first excited states form a well-isolated two-level system. On the other hand, a larger ratio r may lower the sensitivity of the qubit 102 to charge noise. The normal state resistance $R_N$ (and/or the critical current $I_c$) of the tunnel junction and the total capacitance $C_\Sigma$ of the qubit 102 may be used as tuning parameters for achieving a desired energy spacing $E_{01}$ and/or a desired ratio r.

A desired normal state resistance $R_N$ (and/or critical current $I_c$) of the tunnel junction and total capacitance $C_\Sigma$ of the qubit 102 may for example be obtained by choosing the physical dimensions and/or the shape of the superconducting island 104 and/or of the tunnel barrier 108 accordingly. The tunnel barrier 108 may for example have a length L longitudinally across the tunnel barrier 108 (i.e., in a direction from the island 104 to the reservoir 106, e.g., parallel to the X axis of Figs. 1a, 1b), a width w transversally across the tunnel barrier 108 (i.e., in a direction perpendicular to the (longitudinal) direction from the island 104 to the reservoir 106 and, for example, parallel to a surface of the substrate 110, e.g., parallel to the Y axis of Fig. 1a) and a height/thickness h vertically across the tunnel barrier 108 (i.e., in a direction perpendicular to the longitudinal and transversal direction and, for example, perpendicular to a surface of the substrate 110, e.g., parallel to the Z axis of Fig. 1b).

The height h of the tunnel barrier 108 (which may, e.g., correspond to a thickness of a superconducting film that the qubit 102 was formed of) may for example be between 5 nm and 200 nm, preferably between 10 nm and 100 nm, in some examples between 20 nm and 50 nm. The width w of the tunnel barrier 108 (which may, e.g., correspond to a width of the island 104 and/or of the reservoir 108 or may be smaller, in some examples substantially smaller than one or both of the width of the island 104 and the width of the reservoir 108, e.g., to form a physical constriction as illustrated in Figs. 3a, 3b) may for example be between 5 nm and 2000 nm, preferably between 10 nm and 1000 nm, most preferably between 50 nm and 600 nm, in some examples between 100 nm and 500 nm. A cross-sectional area of the tunnel barrier 108 (e.g., the product of the width w and the height h in case of a tunnel barrier 108 having a rectangular cross-section) may for example be between $0.5 \cdot 10^3$ nm$^2$ and $2.0 \cdot 10^4$ nm$^2$, preferably between $1.0 \cdot 10^3$ nm$^2$ and $1.5 \cdot 10^4$ nm$^2$, in some examples between $2.0 \cdot 10^3$ nm$^2$ and $1.0 \cdot 10^4$ nm$^2$. The length L of the tunnel barrier 108 may for example be between 0.5 nm and 50 nm, preferably between 1 nm and 25 nm, most preferably between 5 nm and 15 nm. The length L of the tunnel barrier 108 may be on the same order of magnitude as the coherence length $\xi_0$ of the superconducting material (e.g., $\xi_0 \approx 2.5$ nm for NbTiN).

The physical dimensions and/or the shape of the superconducting island 104 and/or of the tunnel barrier 108 may for example be chosen such that one or more of:

- the normal state resistance $R_N$ of the tunnel junction is between 100 $\Omega$ and 2 k$\Omega$, in some examples between 200 $\Omega$ and 1 k$\Omega$, in one example between 300 $\Omega$ and 500 $\Omega$; and/or

- the total capacitance $C_\Sigma$ of the superconducting qubit 102 is between 10 fF and 1000 fF, in some examples between 20 fF and 500 fF, in one example between 40 fF and 200 fF and in one example between 50 fF and 100 fF; and/or

- the critical current $I_c$ of the tunnel junction is between 1 nA and 1.0 $\mu$A, in some examples between 5 nA and 0.5 $\mu$A, in one example between 10 nA and 0.1 $\mu$A.

The ratio with $r = E_J/E_C$ of the Josephson energy and the charging energy may for example be between 100 and 1 000, preferably between 200 and 600, in some examples between 300 and 500, e.g., 400. This may allow for making the qubit 102 robust against charge noise while still maintaining a suitable degree of anharmonicity. The Josephson energy $E_J$ may for example be between 20 GHz and 2 THz, in some examples between 50 GHz and 500 GHz, in one example between 100 GHz and 300 GHz. The charging energy $E_C$ may for example be between 50 MHz and 5 GHz, in some examples between 0.1 GHz and 1.0 GHz, in one example between 0.3 GHz and 0.7 GHz. The energy splitting $E_{01}$ between the ground state and the first excited state of the qubit 102 may for example be between 10 GHz and 100 GHz, preferably between 20 GHz and 50 GHz, in one example between 25 GHz and 35 GHz. The detuning between the energy splitting $E_{01}$ of the qubit 102 and the energy difference $E_{12}$ between the first excited state and the second excited state of the qubit 102 may for example be at least 0.5%, preferably at least 1.0%, most preferably at least 1.5% of the energy splitting $E_{01}$ and/or may be at least 0.1 GHz, preferably at least 0.2 GHz, most preferably at least 0.4 GHz.

In some examples, the qubit circuit 100 may be embodied as or arranged on and/or in a chip or a chip package (referred to in short as chip in the following). The qubit circuit 100 and/or the chip may comprise a plurality of qubits and/or a plurality of quantum gates. The qubit circuit 100 and/or the chip may for example comprise at least two qubits (e.g., between 2 and 4096 qubits), in some examples at least 16 qubits (e.g., between 16 and 4096) qubits, in one example at least 64 qubits (e.g., between 64 and 4096 qubits). It is, however, noted that the present invention is not limited to any particular number of qubits and that the qubit circuit 100 and/or the chip may comprise any number of qubits. In some examples, the qubit circuit 100 and/or the chip or chip package may be a quantum processor and/or a quantum sensor.

Fig. 2 shows a flow diagram of a method 200 of forming a superconducting qubit comprising a tunnel junction according to the second aspect of the present invention in accordance with an example. The method 200 may for example be used to form a superconducting qubit circuit (or the qubit thereof) according to the first aspect of the present invention in accordance with any one of the embodiments described herein, for example any one of the superconducting qubit circuits 100, 300 and 400. In the following, the superconducting qubit circuit 100 of Figs. 1a, 1b is used as a non-limiting example for illustration purposes. The method 200 is not limited to the order of execution implied by the flow diagram of Fig. 2. As far as technically feasible, the method 200 may be executed in an arbitrary order and steps thereof may be executed simultaneously as least in part. For example, the disorder-induced tunnel barrier in the superconducting material created in step 204 may be created prior to, while and/or after forming the superconducting structure of the superconducting qubit in step 202.

The method 200 comprises, in step 202, forming a superconducting structure of the superconducting qubit 102 such as, for example, some or all of the superconducting island 104, the superconducting reservoir 106 and a region therebetween for the tunnel barrier 108. The superconducting structure is formed of a superconducting material having a critical temperature above the critical temperature of aluminum, i.e., above 1.2 K. The superconducting structure may be formed above (e.g., on) a substrate such as the substrate 110, which may for example be an insulating substrate such as an MgO substrate, a silicon substrate or a sapphire substrate. In one example, the superconducting material is a nitride-based superconductor such as NbTiN, NbN and/or TiN. The superconducting structure may for example be formed by patterned deposition, e.g., using a suitable mask, and/or by deposition of a superconducting film and subsequent patterning thereof, e.g., as described below for method 500 of Fig. 5.

The method 200 further comprises, in step 204, forming a tunnel junction of the superconducting qubit by creating a disorder-induced tunnel barrier such as the tunnel barrier 108 in the superconducting structure, e.g., in the region of the superconducting structure arranged between the island 104 and the reservoir 106. The tunnel barrier 108 is created by introducing spatial crystallographic defects into the superconducting material, e.g., as described above for the superconducting qubit circuit 100.

Creating the spatial crystallographic defects in the superconducting material may for example comprise irradiating the superconducting material with ions, in particular noble gas ions such as helium ions and/or neon ions. Preferably, a focused ion beam is used for this, which may offer improved control over the physical dimensions and/or the shape of the tunnel barrier 108, in particular its length L. The focused ion beam may for example be generated by an ion microscope, in particular a helium ion microscope and/or a neon ion microscope.

The radiation dose may be chosen depending on the desired amount of spatial crystallographic defects, e.g., by adjusting an intensity of the ion beam and/or an exposure time accordingly. The radiation dose may in particular be chosen based on a thickness of the superconducting material, e.g., the height h of the tunnel barrier 108. The superconducting material within the tunnel barrier 108 may for example be irradiated with a dose of between $10^{18}$ ions/cm² and $10^{20}$ ions/cm² with the height h being between 10 nm and 100 nm, in some examples with a dose of between $5 \cdot 10^{18}$ ions/cm² and $5 \cdot 10^{19}$ ions/cm² with the height h between 20 nm and 50 nm, e.g., a dose of $1.0 \cdot 10^{19}$ ions/cm² at a height h of between 30 nm and 40 nm.

The kinetic energy of the ions may be chosen such that the ions (at least predominantly) are not stopped within the superconducting material/the tunnel barrier 108 (e.g., when irradiating along the z direction of Fig. 1b) but penetrate through the superconducting material/the tunnel barrier 108 to reach the substrate 110, for example to avoid implanting impurities into the superconducting material or at least reduce the number thereof. The kinetic energy may for example also be chosen based on the thickness of the superconducting material/the height h of the tunnel barrier 108. The kinetic energy of the ions may for example be between 5 keV and 200 keV with the height h being between 10 nm and 100 nm, in some examples between 10 keV and 100 keV with the height h between 20 nm and 50 nm, in one example of between 20 keV and 40 keV (e.g., 30 keV) at a height h of between 30 nm and 40 nm.

In addition to steps 202 and 204, the method 200 may comprise further steps such as for example coupling the qubit 102 to a readout electrode and/or circuit, a control electrode and/or circuit and/or a flux bias line and/or circuit, for example as described below for method 500 of Fig. 5. In some examples, the method 200 may further comprise connecting the aforementioned circuit elements to external control and/or readout circuitry and/or devices such as a current source, a microwave generator and/or a network analyzer.

Figs. 3a, 3b depict schematic illustrations of a superconducting qubit circuit 300 according to the first aspect of the present invention in accordance with another example. Fig. 3a shows the superconducting qubit circuit 300 in top view with Fig. 3b showing a close-up top view of the SQUID-based charge qubit 102 of the superconducting qubit circuit 300.

The superconducting qubit circuit 300 (referred to in short as the qubit circuit 300) is similar to the superconducting qubit circuit 100 of Figs. 1a, 1b. The superconducting qubit circuit 300 comprises a superconducting qubit 102 (referred to in short as the qubit 102) with a pair of tunnel junctions in an elevated-temperature superconducting material, wherein the tunnel junctions are formed by disorder-induced tunnel barriers 108A, 108B with the disorder being created by spatial crystallographic defects in the superconducting material. The qubit 102, in some examples the entire qubit circuit 300 may be formed using a method according to the second aspect of the present invention according to any one of the embodiments disclosed herein, for example the method 200 of Fig. 2 and/or the method 500 of Fig. 5 described below.

In the example of Figs. 3a, 3b, the qubit 102 is embodied as a SQUID-based charge qubit, namely as a SQUID-based Xmon qubit (as an example of a transmon qubit). The qubit 102 comprises a cross-shaped (or X-shaped) superconducting island 104 and a superconducting reservoir 106, which in this example is formed by a reference electrode 302 such as a ground electrode or ground plate surrounding the superconducting island 104. The island 104 is separated from the reference electrode 302 by an (e.g., cross-shaped) gap, in which no superconducting material is arranged but, for example, an underlying substrate 110 is exposed as illustrated in Fig. 3a. The substrate 110 may for example be embodied as described above for the qubit circuit 100. The island 104 and the reference electrode 302 are weakly coupled via a superconducting quantum interference device (SQUID) 304, which is formed by a superconducting loop comprising a pair of tunnel junctions, each of which is formed by a disorder-induced tunnel barrier 108A and 108B, respectively. Each of the tunnel junctions further comprises a physical constriction, namely a portion of the superconducting loop having a reduced cross-sectional area, for example a reduced width as illustrated in Fig. 3b. A width of the physical constrictions may for example be less than 10%, preferably less than 5%, most preferably less than 1%, in some examples less than 0.5% of a width of the arm of the island 104 that the SQUID 304 is connected to and/or arranged in.

The qubit 102 of the qubit circuit 300 may, at least approximately, be described by a Hamiltonian of the form (see for example J. Koch et al., Phys. Rev. A 76, 042319 (2007))

$$H = 4E_C(\hat{n} - n_g)^2 - E_{J1}(\cos\phi_1) - E_{J2}(\cos\phi_2)$$

with $E_{Ji}$ denoting the Josephson energy of the first/left and second/right tunnel junction, respectively, $\phi_i$ the phase difference across the first/left and second/right tunnel junction, respectively, $E_C$ the charging energy of the superconducting island 104, $\hat{n}$ the number of Cooper pairs on the island 104 and $n_g$ the effective offset charge number. As result of the magnetic flux quantization along the loop of the SQUID 304, the contribution of the Josephson energies may be adjusted by changing the magnetic flux $\Phi$ through the SQUID 304 (i.e., the magnetic flux threading the loop of the SQUID 304). Thereby, the energy levels of the qubit 102 and in particular the energy splitting between the ground state and the first excited state may be tuned (flux tuning):

$$E_{01}(\Phi) = \sqrt{8E_C E_J(\Phi)} - E_C = E_C\left(2\sqrt{2r} - 1\right)$$

with

$$E_J(\Phi) = E_{J0}|\cos(\pi\Phi/\Phi_0)|\sqrt{1 + d^2\tan^2(\pi\Phi/\Phi_0)}$$

and d being a parameter accounting for an asymmetry of the critical currents.

The parameters and/or physical dimensions of the qubit 102 and the tunnel barriers 108A, 108B may for example be chosen similar as described above for the qubit circuit 100 of Figs. 1a, 1b. The total capacitance of the island 104 and thus the charging energy $C_\Sigma$ may be controlled by choosing the physical dimensions and/or the shape of the island 104 and the surrounding reference electrode 302 and/or the spacing therebetween accordingly. A length of each of the arms of the cross-shaped island 104 may for example be between 10 $\mu$m and 2000 $\mu$m, in some examples between 20 $\mu$m and 500 $\mu$m, in one example between 100 $\mu$m and 200 $\mu$m. A width of each of the arms of the cross-shaped island 104 (which may for example be equal to a width or diameter of the loop of the SQUID 304) may for example be between 2 $\mu$m and 200 $\mu$m, in some examples between 5 $\mu$m and 100 $\mu$m, in one example between 10 $\mu$m and 30 $\mu$m.

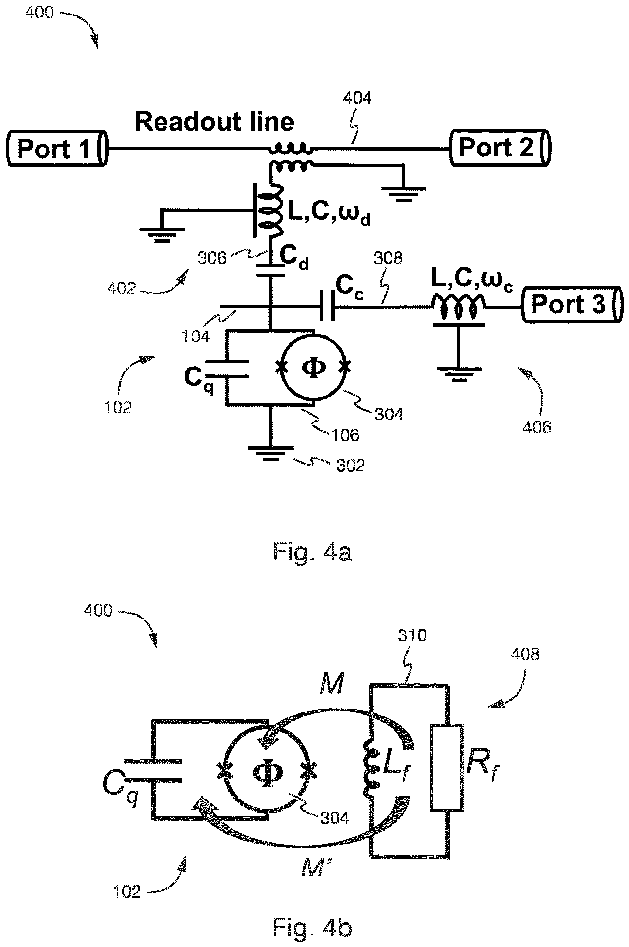

The qubit circuit 300 further comprises a readout electrode 306 for reading out a state of the qubit 102 as well as a control electrode 308 for manipulating a state of the qubit 302. The readout electrode 306 and the control electrode 308 are both capacitively coupled to a respective one of the arms of the island 104. The readout electrode 306 may be coupled to and/or comprised in a readout circuit, for example a transmission readout circuit with a readout resonator, e.g., as in the qubit circuit 400 of Figs. 4a, 4b described below. The control electrode 308 may be coupled to and/or comprised in a control circuit, e.g., similar to the qubit circuit 400 of Figs. 4a, 4b described below. In some examples, the control circuit may couple the qubit 102 to one or more other qubits and/or to one or more quantum gates.

The qubit circuit 300 also comprises a flux bias line 310. The flux bias line 310 may be inductively coupled to the SQUID 304 and/or the qubit 102. The flux bias line 310 may be configured to generate a magnetic field or magnetic flux through the SQUID 304. The magnetic field strength or magnetic flux may be controlled by adjusting a current through the flux bias line 310, e.g., for flux tuning of the energy splitting of the qubit 102. The flux bias line 310 may be coupled to and/or comprised in a flux bias circuit, e.g., similar to the qubit circuit 400 of Figs. 4a, 4b described below.

Some or preferably all of the structures shown in Figs. 3a, 3b, i.e., the qubit 102 with the island 104, the reservoir 106 and the SQUID 304 with the tunnel barriers 108A, 108B, the reference electrode 302, the readout electrode 306, the control electrode 308 and/or the flux bias line 310, maybe made of the same elevated-temperature superconducting material. In one example, the superconducting material is a nitride-based superconductor such as NbTiN. The respective structures may in particular be formed of a same film of this elevated-temperature superconducting material, which may for example be arranged on a substrate 110 similar as described above for the qubit circuit 100 of Figs. 1a, 1b. The respective structures may for example be formed by patterning the film, e.g., as described below for method 500 of Fig. 5.

Figs. 4a depicts a circuit diagram of a superconducting qubit circuit 400 according to the first aspect of the present invention in accordance with another example. Fig. 4b depicts a circuit diagram of a flux bias circuits 408 for and/or of the superconducting qubit circuit 400 (referred to in short as qubit circuit 400).

The qubit circuit 400 is similar to the qubit circuit 100 of Figs. 1a, 1b and the qubit circuit 300 of Figs. 3a, 3b. The qubit circuit 400 also comprises a SQUID-based superconducting charge qubit 102 (referred to in short as qubit 102) with a SQUID 304 arranged between a superconducting island 104 and a superconducting reservoir 106/ground plate 302, a readout electrode 306, a control electrode 308 and a flux bias line 310, all of which may, for example, be embodied as in the qubit circuit 100 and/or in the qubit circuit 300. The island 104 is weakly coupled to the reservoir 106/the ground plate 302 via a pair of tunnel junctions in the SQUID 304. In parallel, the island 104 is also capacitively coupled to the reservoir 106/the ground plate 302 via an additional shunt capacitance $C_q$, which as described above may for example be tailored by choosing the physical dimensions and/or the shape of the island 104 appropriately, e.g., to achieve a desired ratio $E_J/E_C$.

The readout electrode 306, which is capacitively coupled to the island 104 of the qubit 102 with a readout capacitance (diagnostic capacitance) $C_d$, is comprised in a readout circuit 402. The readout circuit 402 is coupled to (and/or comprises) a readout line 404, which is embodied as a transmission readout line in this example. The readout circuit 402 comprises a diagnostic resonator, which is embodied as a $\lambda/4$ resonator in this example. The readout capacitance $C_d$ may determine a coupling strength between the island 304 and the readout electrode 306/the readout circuit 402, e.g., a rate at which photons can exchange between the qubit 102 and the diagnostic resonator. The readout capacitance $C_d$ may thus determine the change $\chi$ in the resonator frequency associated with a transition between the qubit states, e.g., as detailed below. The readout circuit 402 may be coupled inductively to the readout line 404, e.g., at a current node of the diagnostic resonator. The readout circuit 402 may for example be implemented as described in Koch et al., Phys. Rev. A 76, 042319 (2007), R. Barends et al., Phys. Rev. Lett. 111, 080502 (2013) and/or A. Wallraff et al., Nature 431, 162-167 (2004).

As a result of the coupling of the qubit 102 to the readout circuit 402, a resonator frequency of the diagnostic resonator may depend on the state of the qubit 102. A transition between the qubit states may for example change the resonator frequency by a dispersive shift $+\chi$ (or $-\chi$). A coupling strength between the qubit 102 and the resonator may for example be chosen such that the dispersive shift $\chi$ is between 2 and 10 times, preferably between 3 and 5 times the linewidth (Q-factor) of the resonator. Additionally or alternatively, the frequency shift $\chi$ may for example be between 0.5 MHz and 100 MHz, preferably between 1 MHz and 10 MHz, in some examples between 3 MHz and 8 MHz. This may for example be achieved by choosing a ratio (voltage divider) $\beta$ between the readout capacitance $C_d$ and the total capacitance $C_\Sigma$ of the qubit 102, wherein $\beta$ may for example be between 0.05 and 0.2. In some examples, the dispersive shift may scale (at least approximately) as

$$\hbar\chi \approx -(\beta e\, V_{rms}^0)^2 \left(\frac{E_J}{2E_C}\right)^{1/2} \frac{E_C}{\hbar\Delta_0\,(\hbar\Delta_0 - E_C)}$$

with $V_{rms}^0$ denoting the root-mean-square voltage on the resonator associated with one photon (e.g., microwave photon), e the elementary charge and $\Delta_0$ the qubit-resonator detuning, cf. Koch et al., Phys. Rev. A 76, 042319 (2007). The resonator may be detuned from the energy splitting of the qubit 102, in particular with a negative detuning (such that the resonator frequency is higher than the frequency associated with the energy splitting $E_{01}$ of the qubit 102). The detuning $\Delta_0$ may for example be between 1% and 20%, preferably between 2% and 15%, most preferably between 5% and 10% of the energy splitting $E_{01}$ and/or may be between 0.5 GHz and 5 GHz, preferably between 1 GHz and 3 GHz. The value of the readout capacitance $C_d$ may for example be adjusted (in particular in relation to the total capacitance $C_\Sigma$ to adjust the ratio $\beta$) to have a desired dispersive shift between the qubit and the resonator at a given detuning. In a preferred embodiment, the qubit circuit 400 is operated in the so-called "straddling regime" with $0 < \Delta_0 < E_C$, in which large dispersive shifts may be achieved, see also Koch et al., Phys. Rev. A 76, 042319 (2007).

The control electrode 308, which is capacitively coupled to the island 104 of the qubit 102 with a capacitance $C_c$, is comprised in a control circuit 406, which may for example be used to manipulate a state of the qubit 102, e.g., to excite the qubit 102. Additionally or alternatively, the control electrode 308 (or preferably an additional control electrode capacitively coupled to the left arm of the island 104, which is not shown in Figs. 3a and 4a for the sake of simplicity) may be used to manipulate the state of the qubit 102 by coupling the qubit 102 to one or more other qubits and/or one or more quantum gates (e.g., by capacitive coupling to a quantum bus resonator).

The qubit circuit 400 may further comprise a flux bias circuit 408 with the flux bias line 310. The flux bias line 310 may be inductively coupled to the qubit 102, for example with a mutual inductance M to the SQUID 304 and with a mutual inductance M' to the entire qubit 102 (i.e., the entire circuit including the shunt capacitance $C_q$). A ratio of the inductances M' and M, i.e., M'/M, may for example be between 0.01 and 0.3, preferably between 0.02 and 0.2, in some examples between 0.05 and 0.15. The inductance M to the SQUID 304 may for example be between 0.5 pH and 10 pH, in some examples between 1.0 pH and 3.0 pH.

Fig. 5 shows a flow diagram of a method 500 of forming a superconducting qubit comprising a tunnel junction according to the second aspect of the present invention in accordance with another example. The method 500 may for example be used to form a superconducting qubit circuit (or the qubit thereof) according to the first aspect of the present invention in accordance with any one of the embodiments described herein, for example any one of the superconducting qubit circuits 100, 300 and 400. In the following, the superconducting qubit circuits 300 and 400 are used as non-limiting examples for illustration purposes. The method 500 is not limited to the order of execution implied by the flow diagram of Fig. 5. As far as technically feasible, the method 500 may be executed in an arbitrary order and steps thereof may be executed simultaneously as least in part. For example, the disorder-induced tunnel barrier in the superconducting material created in step 506 may be created prior to, while and/or after forming the elements of the superconducting qubit circuit in steps 504 and 506.

The method 500 comprises, in step 502, forming a film, in particular a thin film, of an elevated-temperature superconducting material such as, for example NbTiN, e.g., $Nb_{0.62}Ti_{0.38}N$, on a substrate such as the substrate 110. The substrate 110 may be an insulating substrate, for example an MgO substrate, preferably a crystalline MgO substrate having a well-defined crystallographic orientation, e.g., a cubit (100)-oriented MgO substrate. The film may for example be deposited on the substrate 110 by physical vapor deposition, e.g., pulsed laser deposition. For forming an NbTiN film, NbTi targets may for example be ablated by a laser, e.g., a pulsed infrared laser, in a nitrogen atmosphere, preferably an ultrahigh purity nitrogen atmosphere. The film may for example be deposited at a rate of between 0.1 nm/min and 10 nm/min, preferably of between 0.5 nm/min and 2 nm/min. The film may be deposited with a thickness of between 5 nm and 200 nm, preferably of between 10 nm and 100 nm, in one example of between 20 nm and 50 nm. The film may be deposited with a uniform thickness, wherein the thickness may for example vary by less than 20%, preferably by less than 10% throughout the film. The critical temperature of NbTiN may vary with the thickness of the film and may, e.g., be about 11 K at a thickness of 20 nm, 15 K at a thickness of 50 nm and 16 K at a thickness of 100 nm.

In step 504, a mask (e.g., an etching mask) for forming a superconducting structure of the qubit and/or qubit circuit is formed on the superconducting film. The mask may for example be used for forming some or all of the elements of the qubit circuit 300 or 400, e.g., may be used for forming the superconducting island 104, the superconducting reservoir 106, the reference electrode/ground plate 302, the SQUID 304 (in some examples including the physical constrictions for the tunnel junctions), the readout electrode 306, the control electrode 308 and/or the flux bias line 310. In some examples, the mask may also be used for forming some or all of the readout circuit 402, the readout line 404, the control circuit 406 and the flux bias circuit 408 at least in part, in some examples in their entirety.

The mask may for example be formed by lithography, in particular electron beam lithography. A resist, e.g., a positive or a negative resist, may be formed on the superconducting film, e.g., by spin coating. The resist may then be patterned by lithography and developed. In some examples, different resists may be used for forming the mask, for example a first resist for forming larger features (e.g., the island 104, the ground plate 302, the readout electrode 306, the control electrode 308 and/or the flux bias line 310) and a second resist different from the first resist for forming smaller features (e.g., the SQUID 304 with the tunnel junction portions and/or capacitive elements between the island 104 and the readout electrode 306, the control electrode 308 and/or the flux bias line 310). The first resist may for example be a low-resolution resist configured for a lithographic resolution of between 10 nm and 50 nm (e.g., an AR-N 7520 E-Beam resist by Allresist GmbH) and the second resist may be a high-resolution resist configured for a lithographic resolution of between 1 nm and 10 nm (e.g., an AR-P 6200 E-beam resist by Allresist GmbH).

In step 506, the superconducting film is patterned using the mask to form the superconducting structure of the qubit and/or qubit circuit, e.g., the aforementioned elements of the qubit circuit 300 or 400. The superconducting film may for example be patterned by etching, in particular reactive ion etching (e.g., in fluorine plasma), using the mask as an etching mask. The etching speed may for example be between 2 nm/min and 100 nm/min, preferably between 5 nm/min and 30 nm/min. Subsequently, the mask may be removed. In some examples, step 506 may also comprise a milling of the superconducting film, in particular an ion milling such as argon ion milling.

In step 508, the tunnel junctions for the SQUID 304 are formed by creating the disorder-induced tunnel barrier 108A, 108B in the superconducting structure/film. The tunnel barriers 108A, 108B are created by introducing spatial crystallographic defects into the superconducting material, for example by irradiation with noble gas ions, in this example by direct writing of the tunnel barriers 108A, 108B with a focused helium ion beam. For this, a helium ion microscope may be used, wherein a resolution of the helium ion microscope preferably is below 2.0 nm, most preferably between 1.0 nm, e.g., between 0.2 nm and 1.0 nm. The helium ions may for example have a kinetic energy of between 20 keV and 40 keV, in some examples of between 25 keV and 35 keV, e.g., 30 keV. The area of the tunnel barriers 108A, 108B may, e.g., be irradiated with a dose as described above, e.g., with a dose of between $5 \cdot 10^{18}$ ions/cm$^2$ and $5 \cdot 10^{19}$ ions/cm$^2$. The dose may for example be chosen such that the tunnel junctions are superconducting-normal-superconducting (SNS) or superconducting-insulating-superconducting (SIS) tunnel junctions. The tunnel barriers 108A, 108B may for example be formed with a length of between two and six times the coherence length of the superconducting material, which may, e.g., be about 2.5 nm for $Nb_{0.62}Ti_{0.38}N$.

The embodiments of the present invention disclosed herein only constitute specific examples for illustration purposes. The present invention can be implemented in various ways and with many modifications without altering the underlying basic properties. Therefore, the present invention is only defined by the claims as stated below.

**Claims**

1.  A superconducting qubit circuit (100, 300, 400) with a superconducting qubit (102), wherein said superconducting qubit (102) comprises a tunnel junction in a superconducting material having a critical temperature above 1.2 K, said tunnel junction being formed by a disorder-induced tunnel barrier (108, 108A, 108B), wherein said disorder is created by spatial crystallographic defects in said superconducting material.

2.  The superconducting qubit circuit (100, 300, 400) of claim 1, wherein said superconducting material has a critical temperature of at least 4.2 K, preferably of at least 10 K and/or wherein said superconducting material has a superconducting energy gap of more than 0.2 meV, preferably of at least 1.0 meV, most preferably of at least 2.0 meV.

3.  The superconducting qubit circuit (100, 300, 400) of claim 1 or 2, wherein said superconducting qubit circuit (100, 300, 400) is for operation at an operating temperature of at least 1.0 K, preferably of at least 1.5 K, most preferably of at least 2.0 K.

4.  The superconducting qubit circuit (100, 300, 400) of any one of the preceding claims, wherein said superconducting material comprises a nitride-based superconductor, in particular one or more of niobium titanium nitride (NbTiN), niobium nitride (NbN) and titanium nitride (TiN), and/or wherein said superconducting material comprises one or more of yttrium barium copper oxide (YBCO), magnesium diboride (MgB$_2$), bismuth strontium calcium copper oxide (BSCCO) and an iron-based superconductor.

5.  The superconducting qubit circuit (100, 300, 400) of any one of the preceding claims, wherein:

    the spatial crystallographic defects in said tunnel barrier (108, 108A, 108B) were created by irradiation with noble gas ions, in particular helium ions and/or neon ions, preferably by irradiation with a focused ion beam of noble gas ions; and/or
    said tunnel barrier is free of aluminum oxide, in particular wherein said tunnel junction is free of aluminum.

6. The superconducting qubit circuit (100, 300, 400) of any one of the preceding claims, wherein a cross-sectional area of said tunnel barrier (108, 108A, 108B) is less than $3.0 \cdot 10^4$ nm$^2$, preferably less than $2.0 \cdot 10^4$ nm$^2$, most preferably less than $1.5 \cdot 10^4$ nm$^2$ and/or wherein a length (L) of said tunnel barrier (108, 108A, 108B) is between 0.5 nm and 50 nm, preferably between 1 nm and 25 nm, most preferably between 5 nm and 15 nm.

7. The superconducting qubit circuit (100, 300, 400) of any one of the preceding claims, wherein:

   a normal state resistance of said tunnel junction is at least 100 $\Omega$, preferably at least 200 $\Omega$, most preferably at least 300 $\Omega$; and/or
   a total capacitance of said superconducting qubit is at least 10 fF, preferably at least 20 fF, most preferably at least 50 fF; and/or
   a critical current of said tunnel junction is less than 1.0 $\mu$A, preferably less than 0.5 $\mu$A, most preferably less than 0.2 $\mu$A.

8. The superconducting qubit circuit (100, 300, 400) of any one of the preceding claims, wherein:

   said superconducting qubit (102) is a charge qubit, in particular a SQUID-based charge qubit, wherein a ratio $E_J/E_C$ of a Josephson energy $E_J$ and a charging energy $E_C$ of said charge qubit is between 50 and 1 000, preferably between 200 and 600; and/or
   an energy splitting of said superconducting qubit (102) is between 10 GHz and 100 GHz, preferably between 20 GHz and 50 GHz.

9. The superconducting qubit circuit (100, 300, 400) of any one of the preceding claims, further comprising one or more of a readout electrode (306) and/or a readout circuit (402, 404) for reading out a state of said superconducting qubit (102), a control electrode (308) for manipulating a state of said superconducting qubit (102) and a flux bias line (310) for adjusting the energy splitting of said superconducting qubit (102), in particular wherein said tunnel junction and one or more of, preferably all of said readout electrode (306), said readout circuit (402, 404), said control electrode (308) and said flux bias line (310) are made of said superconducting material, in particular of a same film of said superconducting material.

10. A method (200, 500) of forming a superconducting qubit (102) comprising a tunnel junction, the method (200, 500) comprising:

    forming a superconducting structure (104, 106) of said superconducting qubit (102), said superconducting structure (104, 106) being formed of a superconducting material having a critical temperature above 1.2 K; and
    forming said tunnel junction by creating a disorder-induced tunnel barrier (108, 108A, 108B) in said superconducting structure (104, 106), said tunnel barrier (108, 108A, 108B) being created by introducing spatial crystallographic defects into said superconducting material.

11. The method (200, 500) of claim 10, wherein said spatial crystallographic defects are introduced into said superconducting material by irradiating said superconducting material with noble gas ions, in particular helium ions and/or neon ions, preferably by irradiation with a focused ion beam of noble gas ions.

12. The method (200, 500) of claim 11, wherein said superconducting material is irradiated with a dose of between $10^{17}$ ions/cm$^2$ and $10^{21}$ ions/cm$^2$, preferably of between $10^{18}$ ions/cm$^2$ and $10^{20}$ ions/cm$^2$, and/or wherein said superconducting material is arranged on a substrate (110) and a kinetic energy of said noble gas ions is chosen such that said noble gas ions penetrate through said superconducting material into said substrate (110).

13. The method (200, 500) of any one of claims 10 to 12, wherein forming said superconducting structure (104, 106) comprises forming a film of said superconducting material and patterning said film to form the superconducting structure (104, 106), in particular wherein the method (200, 500) further comprises forming one or more of a readout electrode (306) and/or a readout circuit (402, 404) for reading out a state of said superconducting qubit (102), a control electrode (308) for manipulating a state of said superconducting qubit (102) and a flux bias line (310) for adjusting an energy splitting of said superconducting qubit (102), in particular wherein one or more of, preferably all of said readout line (306), said readout circuit (402, 404), said control line (308) and said flux bias line (310) are formed from said film of said superconducting material.

14. The superconducting qubit circuit (100, 300, 400) of any one of claims 1 to 9, wherein said superconducting qubit (102)

**EP 4 572 590 A1**

is obtainable using a method (200, 500) according to any one of claims 10 to 13.

15. Use of the superconducting qubit circuit (100, 300, 400) of any one of claims 1 to 9 or of claim 14, wherein said superconducting qubit circuit (100, 300, 400) is operated at an operating temperature of at least 1.0 K, preferably of at least 1.5 K, most preferably of at least 2.0 K.

100 — 102

L

w

106    108    104

y

x

110

Fig. 1a

100 — 102

106    L    104

h

110

z

108

x

Fig. 1b

200

form superconducting qubit structure
of high-temperature
superconducting material

202

form tunnel junction by creating
disorder-induced tunnel
barrier in superconducting material

204

Fig. 2

Fig. 3a

Fig. 3b

Fig. 4a

Fig. 4b

500

form superconducting film
on substrate

502

form mask
for qubit circuit elements
on superconducting film

504

form qubit circuit elements by
patterning of superconducting
film with mask

506

create disorder-induced
tunnel barrier by
direct writing with ion beam

508

Fig. 5

Europäisches
Patentamt
European
Patent Office
Office européen
des brevets

# EUROPEAN SEARCH REPORT

Application Number

EP 23 21 6396

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHEN? ZIWEN?? ET AL: "High-Temperature Superconducting YBa2Cu3O7 - [delta] Josephson Junction Fabricated with a Focused Helium Ion Beam", CHINESE PHYSICS LETTERS, INSTITUTE OF PHYSICS PUBLISHING, BRISTOL, GB, vol. 39, no. 7, 6 June 2022 (2022-06-06), XP020433319, ISSN: 0256-307X, DOI: 10.1088/0256-307X/39/7/077402 [retrieved on 2022-06-06] * page 2, right-hand column - page 3, column 8; figures 1, 2 * * page 1, left-hand column, line 4 * | 1-15 | INV. H10N60/01 H10N60/12 G06N10/40 |
| X | KASAEI L ET AL: "MgB2Josephson junctions produced by focused helium ion beam irradiation", AIP ADVANCES, AMERICAN INSTITUTE OF PHYSICS, 2 HUNTINGTON QUADRANGLE, MELVILLE, NY 11747, vol. 8, no. 7, 19 July 2018 (2018-07-19), XP012230182, DOI: 10.1063/1.5030751 [retrieved on 2018-07-19] * page 2, line 27 - page 3, line 13; figure 1 * | 1-11, 13-15 | |

TECHNICAL FIELDS SEARCHED (IPC)

H10N
G06N

The present search report has been drawn up for all claims

| Place of search | Date of completion of the search | Examiner |
|---|---|---|
| Munich | 6 May 2024 | Gröger, Andreas |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 11538977 B2

**Non-patent literature cited in the description**

- **A. RUHTINAS ; I. J. MAASILTA.** *ar-Xiv:2303.17348v1*
- **J. KOCH et al.** *Phys. Rev.*, 2007, vol. 76, 042319
- **R. BARENDS et al.** *Phys. Rev. Lett.*, 2013, vol. 111, 080502
- **KOCH et al.** *Phys. Rev.*, 2007, vol. 76, 042319
- **A. WALLRAFF et al.** *Nature*, 2004, vol. 431, 162-167